# EUROPÄISCHE PATENTSCHRIFT

(11) **EP 3 209 273 B1**
(45) Veröffentlichungstag und Bekanntmachung des Hinweises auf die Patenterteilung: **10.07.2019**
(21) Anmeldenummer: 15787147.6
(22) Anmeldetag: 08.10.2015
(51) Int. Cl.: A61K 8/35, A61K 8/41, A61K 8/49, A61K 8/73, A61Q 17/04, D06M 15/03

(54) **POLYSACCHARID-HALTIGE SONNENSCHUTZMITTEL MIT REDUZIERTER NEIGUNG ZUR TEXTILVERFLECKUNG**
POLYSACCHARIDE-CONTAINING SUNSCREEN HAVING REDUCED TENDENCY TO STAIN TEXTILES
PRODUIT DE PROTECTION SOLAIRE CONTENANT DES POLYSACCHARIDES ET AYANT UNE TENDANCE RÉDUITE À FORMER DES TACHES SUR LES TEXTILES

(30) Priorität: 22.10.2014 DE 102014015554
(43) Veröffentlichungstag der Anmeldung: 30.08.2017
(60) Teilanmeldung: 17202735.1
(73) Patentinhaber: Beiersdorf AG, 20253 Hamburg (DE)
(72) Erfinder: WEINERT, Katrin, 22763 Hamburg (DE); BORCHERS, Kathrin, 21614 Buxtehude (DE); BLECKMANN, Andreas, 22926 Ahrensburg (DE)
(86) Internationale Anmeldenummer: PCT/EP2015/073227
(87) Internationale Veröffentlichungsnummer: WO 2016/062543

(56) Entgegenhaltungen:
- EP-A2- 2 647 366
- WO-A2-2009/077356
- DE-A1-102010 008 320
- US-A1- 2013 309 185

## Beschreibung

Die vorliegende Erfindung betrifft die Verwendung bestimmter Polysaccharide in UV-Filter enthaltenden kosmetischen Zubereitungen, zur Reduzierung der durch die UV-Filter in der Zubereitung hervorgerufenen Verunreinigung und/oder Verfärbung von Textilien sowie zur leichteren Auswaschbarkeit der durch die UV-Filter in der Zubereitung hervorgerufenen Verunreinigung und/oder Verfärbung von Textilien, sowie entsprechende Verfahren zur leichteren Auswaschbarkeit der Verfleckungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien, Flecken und Verfärbungen. Diese Verfärbungen werden vor allem durch nicht-wasserlösliche UVA- und Breitbandfilter hervorgerufen, insbesondere durch die UV-Filter 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin), 4-(tert.-Butyl)-4'-methoxydibenzoylmethan (INCI Butyl Methoxydibenzoylmethane), und (2-[-4-(Diethylamino)-2-hydroxybenzoyl] Benzoesäurehexylester (INCI: Diethylamino Hydroxybenzoyl Hexyl Benzoate). Die Verfleckungen sind durch Waschen mit herkömmlichen Waschmitteln kaum zu entfernen und verstärken sich während des Waschprozesses durch Wechselwirkungen mit Ionen des Waschwassers sogar noch. Das Problem tritt besonders stark bei Zubereitungen mit hohem Lichtschutzfaktor auf.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche UV-A Filter wie 4-(tert.-Butyl)-4'-methoxydibenzoylmethan (INCI Butyl Methoxydibenzoylmethane) und (2-[-4-(Diethylamino)-2-hydroxybenzoyl] Benzoesäurehexylester (INCI: Diethylamino Hydroxybenzoyl Hexyl Benzoate) und Breitbandfilter wie Bis-Ethylhexyloxyphenol Methoxyphenyl Triazine zu entwickeln, welche sich leichter aus den mit der Zubereitung kontaminierten Textilen herauswaschen lassen.

Überraschend gelöst wird die Aufgabe durch die Verwendung von Polysacchariden aus der Gruppe der Gumen in UV-Filter enthaltenden kosmetischen Zubereitungen, zur Reduzierung der durch die UV-Filter in der Zubereitung hervorgerufenen Verunreinigung und/oder Verfärbung von Textilien,
sowie
durch die Verwendung von Polysacchariden aus der Gruppe der Gumen in UV-Filter enthaltenden kosmetischen Zubereitungen, zur leichteren Auswaschbarkeit der durch die UV-Filter in der Zubereitung hervorgerufenen Verunreinigung und/oder Verfärbung von Textilien.

Überraschend gelöst wird die Aufgabe ferner durch ein Verfahren zur Erleichterung der Auswaschbarkeit von UV-Filter enthaltenden kosmetischen Zubereitungen aus Textilien, dadurch gekennzeichnet, dass dem Kosmetikum ein oder mehrere Polysaccharide aus der Gruppe der Gumen zugesetzt werden sowie durch ein Verfahren zur Reduzierung der durch UV-Filter enthaltenden kosmetischen Zubereitungen hervorgerufenen Verfärbung, dadurch gekennzeichnet, dass dem Kosmetikum ein oder mehrere Polysaccharide aus der Gruppe der Gumen zugesetzt werden.

Zwar sind dem Fachmann durchaus Sonnenschutzmittel mit Polysachariden bekannt, doch wurden diese bislang zur Verbesserung der sensorischen Eigenschaften (Puderrohstoff) bei der Anwendung der Produkte *auf der Haut* oder zur Stabilisierung/ Verdickung der Formulierungen eingesetzt. Dass Polysaccharide jedoch einen Einfluss auf die unerwünschte Textilverfleckung haben und insbesondere zu einer leichteren Auswaschbarkeit des Sonnenschutzmittels und seiner UV-Filter aus den Textilen heraus, führen war bisher unbekannt und auch nicht zu erwarten gewesen.

Ferner kennt der Stand der Technik die DE 102010008320 A1, EP 2647366 A2, WO 2009/077356 A2 und US 2013/309185 A1, die ebenfalls nicht den Weg zur vorliegenden Erfindung weisen konnten.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen das erfindungsgemäße Verfahren verwirklicht wird.

Die erfindungsgemäß vorteilhaften Ausführungsformen der vorliegenden Erfindung sind dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 4-(tert.-Butyl)-4'-methoxydibenzoylmethan (INCI Butyl Methoxydibenzoylmethane), und/oder (2-[-4-(Diethylamino)-2-hydroxybenzoyl] Benzoesäurehexylester (INCI: Diethylamino Hydroxybenzoyl Hexyl Benzoate) als verunreinigende und/oder verfärbende UV-Filter enthält.

Es ist erfindungsgemäß bevorzugt, wenn die verwendeten Polysaccharide aus der Gruppe der Gumen ausgewählt werden aus der Gruppe der Verbindungen Welan Gum, Sclerotium Gum und Cellulose Gum.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Für die erfindungsgemäß vorteilhaften Polysaccharide gelten für die einzelnen Stoffe die folgenden Einsatzkonzentrationen als erfindungsgemäß bevorzugt:
Welan Gum wird erfindungsgemäß bevorzugt in einer Konzentration von 0,01 bis 1,5 Gewichts-%, bezogen auf das Gesamtgewicht der Zusammensetzung eingesetzt. Als Welan Gum kann beispielsweise der Rohstoff Collstab W-100 der Firma Colltec vorteilhaft eingesetzt werden. Sclerotium Gum wird erfindungsgemäß bevorzugt in einer Konzentration von 0,01 bis 1,5 Gewichts-%, bezogen auf das Gesamtgewicht der Zusammensetzung eingesetzt. Als Sclerotium Gum kann beispielsweise der Rohstoff Actigum CS 11der Firma Cargill vorteilhaft eingesetzt werden.

Cellulose Gum wird erfindungsgemäß bevorzugt in einer Konzentration von 0,01 bis 1,5 Gewichts-%, bezogen auf das Gesamtgewicht der Zusammensetzung eingesetzt. Als Cellulose Gum kann beispielsweise der Rohstoff Blanose Cellulose Gum der Firma Ashland vorteilhaft eingesetzt werden.

Erfindungsgemäß besonders bevorzugt wird für die erfindungsgemäße Verwendung bzw. das erfindungsgemäße Verfahren Cellulose Gum eingesetzt, wenn die Auswaschbarkeit von 4-(tert.-Butyl)-4'-methoxydibenzoylmethan (INCI Butyl Methoxydibenzoylmethane) erleichtert werden soll (bzw. durch diesen UV-Filter hervorgerufene Textilverfleckung reduziert werden soll).

Erfindungsgemäß besonders bevorzugt wird für die erfindungsgemäße Verwendung bzw. das erfindungsgemäße Verfahren Cellulose Gum eingesetzt, wenn die Auswaschbarkeit von 2,4-Bis-{[4-(2-ethyl-hexyloxy)-2-hy-droxy]-phenyl}-6-(4-methoxyphenyl)-1,3,5-triazin (INCI: Bis-Ethylhexyloxyphenol Methoxyphenyl Triazin) erleichtert werden soll (bzw. durch diesen UV-Filter hervorgerufene Textilverfleckung reduziert werden soll).

Erfindungsgemäß vorteilhafte Ausführungsformen der vorliegenden Erfindung sind dadurch gekennzeichnet, dass die Zubereitung eine oder mehrere der Säure- bzw. Salzverbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze und/ oder deren Amin-N-Oxide enthält.

In einem solchen Fall ist es erfindungsgemäß bevorzugt, wenn die Zubereitung eine oder mehrere dieser Säure- bzw. Salzverbindungen in einer Gesamtmenge von 0,1 bis 3 Gewichts-%, bezogen auf das Gesamtgewicht der Zubereitung, enthält.

Die der Erfindung zugrunde liegende Aufgabe wird auch gelöst durch eine kosmetische Zubereitung enthaltend eine Kombination aus
a) 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und
b) Welan Gum.

Sie wird darüber hinaus gelöst durch eine kosmetische Zubereitung enthaltend eine Kombination aus
a) 4-(tert.-Butyl)-4'-methoxydibenzoylmethan (INCI Butyl Methoxydibenzoylmethane) und
b) Welan Gum.

Die der Erfindung zu Grunde liegende Aufgabe wird nicht zuletzt gelöst durch eine kosmetische Zubereitung enthaltend eine Kombination aus
a) (2-[-4-(Diethylamino)-2-hydroxybenzoyl] Benzoesäurehexylester (INCI: Diethylamino Hydroxybenzoyl Hexyl Benzoate) und
b) Welan Gum.

Diese kosmetischen Zubereitungen zeichnen sich vorteilhaft dadurch aus, dass die kosmetische Zubereitung dadurch gekennzeichnet ist, dass die Gesamtmenge an Welan Gum und Sclerotium Gum (soweit enthalten) in der Zubereitung von 0,01 bis 2 Gewichts-%, bezogen auf das Gesamtgewicht der Zubereitung beträgt.

Grundsätzlich ist es bei der erfindungsgemäßen Verwendung, dem erfindungsgemäßen Verfahren und der erfindungsgemäßen Zubereitung bevorzugt, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in einer Menge von 0,01 bis 10 Gewichts-%, bezogen auf das Gesamtgewicht der Zubereitung, enthält.

Außerdem ist es grundsätzlich bei der erfindungsgemäßen Verwendung, dem erfindungsgemäßen Verfahren und der erfindungsgemäßen Zubereitung bevorzugt, wenn die Zubereitung 4-(tert.-Butyl)-4'-methoxydibenzoylmethan (INCI Butyl Methoxydibenzoylmethane)
in einer Menge von 0,01 bis 10 Gewichts-%, bezogen auf das Gesamtgewicht der Zubereitung, enthält.

Nicht zuletzt ist es grundsätzlich bei der erfindungsgemäßen Verwendung, dem erfindungsgemäßen Verfahren und der erfindungsgemäßen Zubereitung bevorzugt, wenn die Zubereitung (2-[-4-(Diethylamino)-2-hydroxybenzoyl] Benzoesäurehexylester (INCI: Diethylamino Hydroxybenzoyl Hexyl Benzoate) in einer Menge von 0,01 bis 10 Gewichts-%, bezogen auf das Gesamtgewicht der Zubereitung, enthält.

Die Zubereitungen, in denen die Erfindung verwirklicht wird, können darüber hinaus erfindungsgemäß vorteilhaft weitere UV-Filter enthalten. Diese werden vorteilhaft gewählt aus der Gruppe der Verbindungen Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist dabei erfindungsgemäß besonders bevorzugt, wenn die Zubereitung frei ist von 3-(4-Methylbenzyliden)-campher und 2-Hydroxy-4-methoxybenzophenon (Oxybenzon).

Erfindungsgemäß vorteilhafte Ausführungsformen können dadurch erhalten werden, dass die Zubereitung Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Es ist erfindungsgemäß vorteilhaft, wenn die erfindungsgemäße Zubereitung Phenoxyethanol und/oder Methylparaben enthält.

Es ist dabei erfindungsgemäß bevorzugt, wenn die Zubereitung frei ist von Propyl- und Butylparaben, 3-Iod-2-propinylbutylcarbamat,

Erfindungsgemäß vorteilhafte Ausführungsformen sind auch dadurch gekennzeichnet, dass die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, Hyaluronsäure, Glycyrrhetinsäure, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin, Panthenol und/oder Licochalcon A, enthält.

Die erfindungsgemäße Zubereitung kann ferner vorteilshaft Glycerin und/oder Ethanol enthalten. In einem solchen Falle ist eine Glycerin-Konzentration von 0,01 bis 10 Gewichts-%, bezogen auf das Gesamtgewicht der Zubereitung, erfindungsgemäß vorteilhaft. Für Ethanol liegt der erfindungsgemäß vorteilhafte Einsatzbereich zwischen 0,01 und 10 Gewichts-%, bezogen auf das Gesamtgewicht der Zubereitung.

Die Ölphase der erfindungsgemäßen Zubereitung kann darüber hinaus noch Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß vorteilhafte Ausführungsformen der vorliegenden Erfindung sind auch dadurch gekennzeichnet, dass die Emulsion Dimethicone und/oder Cyclomethicon enthält.

Erfindungsgemäß besonders bevorzugt ist es, wenn die Ölphase der Zubereitung Di-n-Octylcarbonat (INCI Dicaprylyl Carbonate), Di-Isopropyladipat und/oder Di-n-Butyladipat (INCI Dibutyl Adipate) enthält.

Als polymeren Filmbildner zur Erhöhung der Wasserfestigkeit kann die erfindungsgemäße Zubereitung erfindungsgemäß vorteilhaft Vinylpyrrolidon/Hexadecen Copolymer enthalten. Darüber hinaus ist der Zusatz Tapiokastärke erfindungsgemäß vorteilhaft.

Darüber hinaus kann die erfindungsgemäße Zubereitung die für die Kosmetik üblichen Inhaltsstoffe in den entsprechenden Einsatzkonzentrationen enthalten.

Die erfindungsgemäßen Zubereitungen liegen vorteilhaft als Emulsion, Hydrodispersion oder alkoholische Lösung vor. Erfindungsgemäße bevorzugt ist dabei die Emulsion, insbesondere die öl-in-Wasser-Emulsion (O/W-Emulsion).

Die erfindungsgemäße Zubereitung kann insbesondere vorteilhaft als Tagespflegeprodukt oder Sonnenschutzmittel eingesetzt werden.

### Vergleichsversuch

### Mit dem folgenden Versuch konnte der erfindungsgemäße Effekt beispielhaft belegt werden:

Es wurde jeweils 1% der erfindungsgemäßen Polysaccharide zu einer Butyl Methoxydibenzoylmethane oder Bis-Ethylhexyloxyphenol Methoxyphenyl Triazin enthaltenden Formulierung zugesetzt und die verfleckungsreduzierende Wirkung (Reduktion b*) im Vergleich zu einer Formulierung ohne erfindungsgemäße Polysaccharide mittels beschriebener Methode bestimmt. Als Beleg der verbesserten Auswaschbarkeit und verminderten Fleckenbildung der erfindungsgemäßen Zubereitungen wurden in vitro Untersuchungen durchgeführt, deren Ergebnisse in den Tabellen 1 und 2 dargestellt sind.

Es wurden verschiedene Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, zehn Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

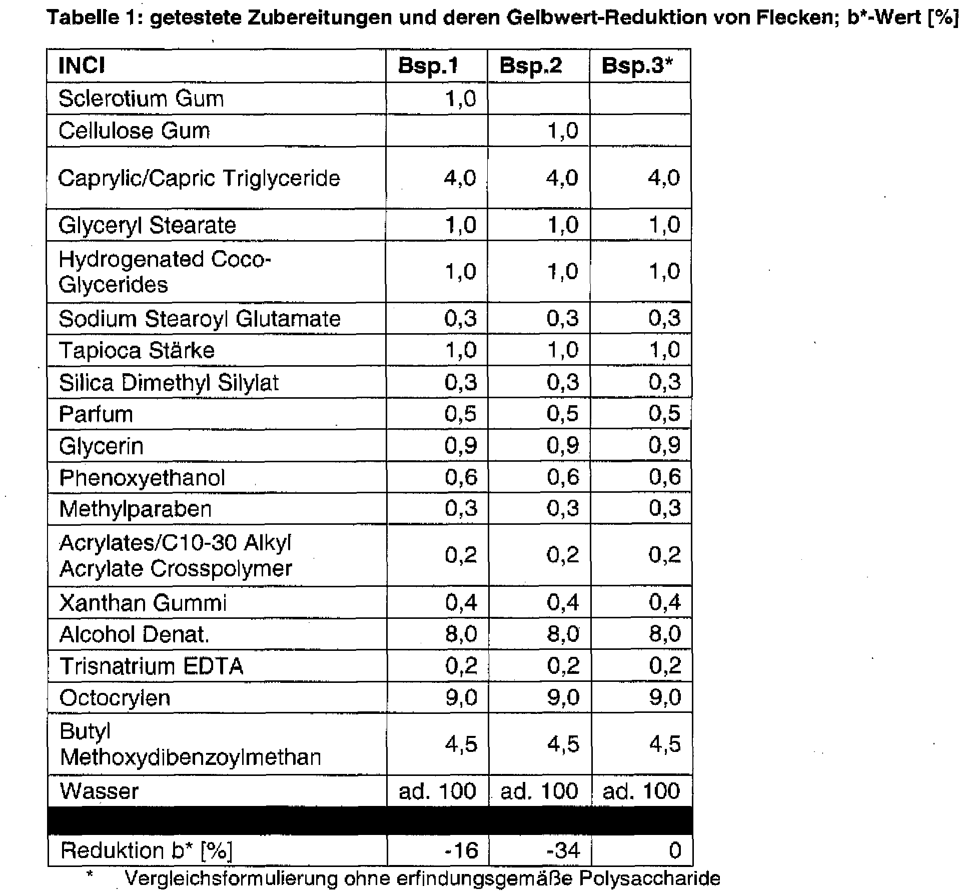

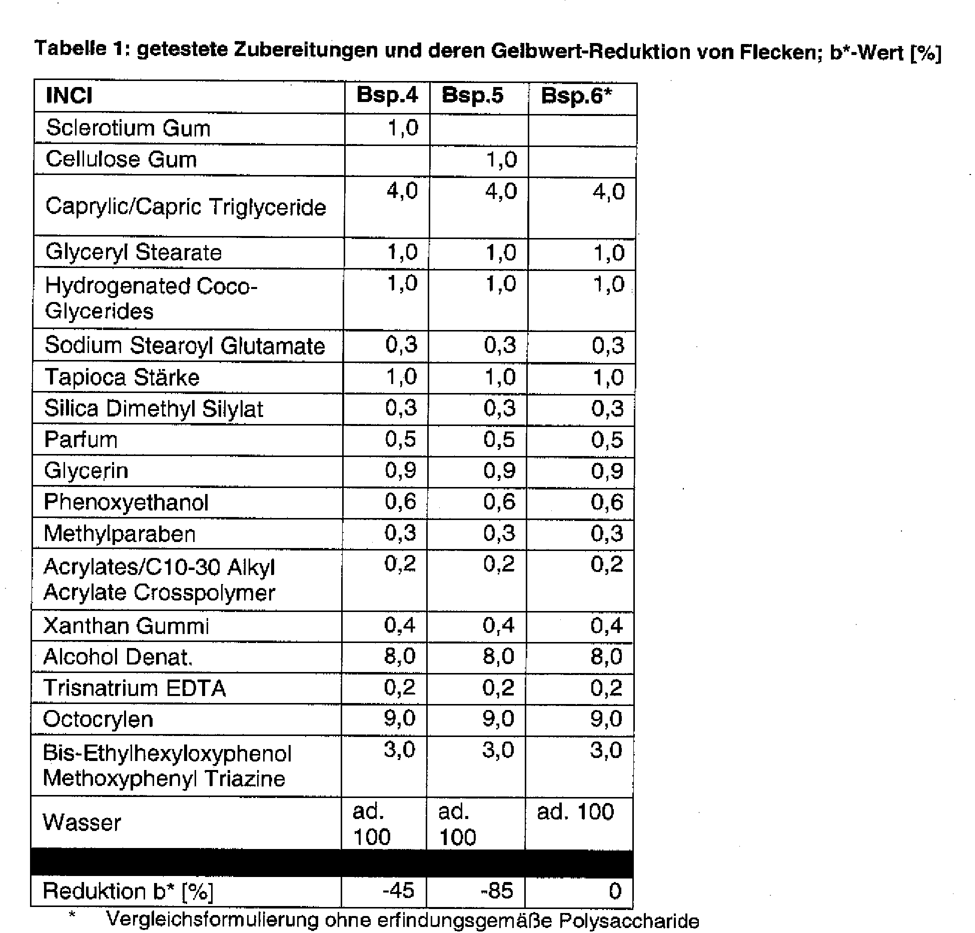

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Tabelle 3: Übersicht Beispielrezepturen**

| **INCI** | | | | | | | |
|---|---|---|---|---|---|---|---|
| | **7** | **8** | **9** | **10** | **11** | **12** | **13** |
| Sclerotium Gum | | | 0,5 | | | | |
| Carrageenan | | | | | | | |
| Carboxymethylcellulose | | 0,75 | 0,5 | | | 0,75 | 0,5 |
| Cellulose Gum | 1,0 | 0,75 | | 1,0 | 1,0 | 0,75 | 0,75 |
| Sodium Alginate | 0,2 | | 0,5 | | | | |
| Trisodium EDTA | 0,20 | 0,50 | 0,50 | 0,50 | 0,20 | 0,20 | 0,50 |
| Tetranatriumiminodisuccinate | | | | | 0,75 | 0,50 | |
| Diethylentriamin-penta(methylenphosphonsäure) | | | | | | 0,50 | 0,50 |
| Butyl methoxydibenzoylmethane | 5,00 | 3,00 | 3,00 | 5,00 | 5,00 | 4,00 | 3,00 |
| Bis-Ethylhexyloxyphenol Methoxyphenyl Triazine | 3,50 | 3,50 | 0,50 | 0,50 | 3,50 | 3,50 | 0,50 |
| Diethylamino Hydroxybenzoyl Hexvlbenzoate | | | | 0,50 | 0,50 | 1,00 | |
| Phenylbenzimidazole Sulfonic Acid | | 1,00 | 1,00 | 1,00 | | 1,00 | 1,00 |
| Ethylhexyl Salicylate | | 5,00 | 5,00 | 5,00 | 4,50 | 5,00 | 5,00 |
| Titanium Dioxide | 3,00 | 2,00 | | | 3,00 | 2,00 | |
| Trimethoxycaprylylsilane | 0,20 | 0,20 | | | 0,20 | 0,20 | |
| Octocrylene | 10,00 | 10,00 | 10,00 | 10,00 | 10,00 | 10,00 | 10,00 |
| Homosalate | | 10,00 | 10,00 | 10,00 | 4,50 | 10,00 | 10,00 |
| Cetearyl Alcohol | | 1,00 | 0,50 | | | 1,00 | 0,50 |
| Xanthan Gum | 0,40 | 0,40 | 0,40 | | 0,40 | 0,40 | 0,40 |
| Acrylates/C10-30 Alkyl Acrylate Crosspolymer | 0,30 | 0,05 | 0,20 | 0,40 | 0,30 | 0,05 | 0,20 |
| Alcohol Denat. | 5,00 | 4,00 | 6,00 | 6,00 | 5,00 | 4,00 | 6,00 |
| Methyl paraben | 0,30 | 0,30 | 0,30 | 0,30 | 0,30 | 0,30 | 0,30 |
| Phenoxyethanol | 0,60 | 0,60 | 0,60 | 0,60 | 0,60 | 0,60 | 0,60 |
| Citric Acid | 0,30 | | | | 0,30 | | |
| Sodium Citrate | 0,10 | | | | 0,10 | | |
| Sodium Hydroxide | 0,20 | 0,40 | 0,50 | 0,70 | 0,20 | 0,40 | 0,50 |
| Glycerin | 3,00 | 9,00 | 3,00 | 9,00 | 3,00 | 9,00 | 3,00 |
| Parfum | 0,40 | 0,40 | 0,60 | 0,30 | 0,50 | 0,40 | 0,60 |
| VP/Hexadecene Copolymer | 0,50 | 0,50 | | 0,50 | 0,50 | 0,50 | |
| Silica Dimethyl Silylate | | 0,50 | 0,50 | | | 0,50 | 0,50 |
| Sodium Cetearyl Sulfate | | 0,15 | | | | 0,15 | |
| Glyceryl Stearate SE | | 1,00 | | | | 1,00 | |
| Glyceryl Stearate Citrate | 2,00 | | | | 2,00 | | |
| Ceteareth-20 | | | | 1,00 | | | |
| Sodium Stearoyl Glutamate | | | 0,40 | | | | 0,40 |
| Glyceryl Stearate | | | 1,00 | | | | 1,00 |
| Hydrogenated Coco-Glycerides | 1,00 | | 1,00 | | 1,00 | | 1,00 |
| C12-15 Alkyl Benzoate | 5,00 | | | | 5,00 | | |
| Myristyl Myristate | 1,00 | 1,00 | | | 1,00 | 1,00 | |
| Stearyl Alcohol | 0,50 | | | | 0,50 | | |
| C18-36 Acid Triglyceride | | | | 0,50 | | | |
| C18-38 Alkyl Hydroxystearoyl Stearate | | 0,50 | | | | 0,50 | |
| Isopropyl Stearate | 2,00 | | | | 2,00 | | |
| Butylene Glycol Dicaprylate/Dicaprate | 5,00 | | | 3,00 | 5,00 | | |
| Aqua | ad 100 | ad 100 | ad 100 | ad 100 | ad 100 | ad 100 | ad 100 |

## Patentansprüche

1. Verwendung von Polysacchariden aus der Gruppe der Gumen in UV-Filter enthaltenden kosmetischen Zubereitungen, zur Reduzierung der durch die UV-Filter in der Zubereitung hervorgerufenen Verunreinigung und/oder Verfärbung von Textilien.

2. Verwendung von Polysacchariden aus der Gruppe der Gumen in UV-Filter enthaltenden kosmetischen Zubereitungen, zur leichteren Auswaschbarkeit der durch die UV-Filter in der Zubereitung hervorgerufenen Verunreinigung und/oder Verfärbung von Textilien.

3. Verfahren zur Erleichterung der Auswaschbarkeit von UV-Filter enthaltenden kosmetischen Zubereitungen aus Textilien, **dadurch gekennzeichnet, dass** dem Kosmetikum ein oder mehrere Polysaccharide aus der Gruppe der Gumen zugesetzt werden.

4. Verfahren zur Reduzierung der durch UV-Filter enthaltenden kosmetischen Zubereitungen hervorgerufenen Verfärbung von Textilien **dadurch gekennzeichnet, dass** dem Kosmetikum ein oder mehrere Polysaccharide aus der Gruppe der Gumen zugesetzt werden.

5. Verwendung oder Verfahren nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 4-(tert.-Butyl)-4'-methoxydibenzoylmethan (INCI: Butyl Methoxydibenzoylmethane), und/oder (2-[-4-(Diethylamino)-2-hydroxybenzoyl] Benzoesäurehexylester (INCI: Diethylamino Hydroxybenzoyl Hexyl Benzoate) als verunreinigende und/oder verfärbende UV-Filter enthält.

6. Verwendung oder Verfahren nach einem der vorhergehenden Ansprüche, **dadurch gekennzeichnet, dass** die verwendeten Polysaccharide ausgewählt werden aus der Gruppe der Verbindungen.Welan Gum, Sclerotium Gum und Cellulose Gum.

7. Verwendung oder Verfahren nach einem der vorhergehenden Ansprüche, **dadurch gekennzeichnet, dass** die Zubereitung Polysaccharide in einer Gesamtmenge von 0,01 bis 2 Gewichts-%, bezogen auf das Gesamtgewicht der Zubereitung, enthält.

8. Verwendung oder Verfahren nach einem der vorhergehenden Ansprüche, **dadurch gekennzeichnet, dass** die Zubereitung eine oder mehrere Säure- bzw. Salzverbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze und/ oder deren Amin-N-Oxide enthält.

9. Verwendung oder Verfahren nach Anspruch 8, **dadurch gekennzeichnet, dass** die Zubereitung eine oder mehrere dieser Säure- bzw. Salzverbindungen in einer Gesamtmenge von 0,1 bis 3 Gewichts-%, bezogen auf das Gesamtgewicht der Zubereitung, enthält.

10. Verwendung oder Verfahren nach einem der vorhergehenden Ansprüche, **dadurch gekennzeichnet, dass** die Zubereitung ein oder mehrere Siloxanelastomere enthält.

11. Verwendung oder Verfahren nach Anspruch 10, **dadurch gekennzeichnet, dass** die Zubereitung eine oder mehrere Siloxanelastomere in einer Gesamtmenge von 0,01 bis 10 Gewichts-%, bezogen auf das Gesamtgewicht der Zubereitung, enthält.

12. Kosmetische Zubereitung enthaltend eine Kombination aus
a) 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und
b) Welan Gum.

13. Kosmetische Zubereitung enthaltend eine Kombination aus
a) 4-(tert.-Butyl)-4'-methoxydibenzoylmethan (INCI Butyl Methoxydibenzoylmethane) und
b) Welan Gum.

14. Kosmetische Zubereitung enthaltend eine Kombination aus
a) (2-[-4-(Diethylamino)-2-hydroxybenzoyl]Benzoesäurehexylester (INCI: Diethylamino Hydroxybenzoyl Hexyl Benzoate) und
b) Welan Gum.

15. Kosmetische Zubereitung nach einem der Ansprüche 12 bis 14, **dadurch gekennzeichnet, dass** die Gesamtmenge an Welan Gum und Sclerotium Gum (soweit enthalten) in der Zubereitung von 0,01 bis 2 Gewichts-%, bezogen auf das Gesamtgewicht der Zubereitung beträgt.

16. Verwendung, Verfahren oder Zubereitung nach einem der vorhergehenden Ansprüche, **dadurch gekennzeichnet, dass** die Zubereitung 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin) in einer Menge von 0,01 bis 10 Gewichts-%, bezogen auf das Gesamtgewicht der Zubereitung, enthält.

17. Verwendung, Verfahren oder Zubereitung nach einem der vorhergehenden Ansprüche 1 bis 11, 13 und 15, **dadurch gekennzeichnet, dass** die Zubereitung 4-(tert.-Butyl)-4'-methoxydibenzoylmethan (INCI Butyl Methoxydibenzoylmethane) in einer Menge von 0,01 bis 10 Gewichts-%, bezogen auf das Gesamtgewicht der Zubereitung, enthält.

18. Verwendung, Verfahren oder Zubereitung nach einem der vorhergehenden Ansprüche 1 bis 11, 14 und 15, **dadurch gekennzeichnet, dass** die Zubereitung (2-[-4-(Diethylamino)-2-hydroxybenzoyl] Benzoesäurehexylester (INCI: Diethylamino Hydroxybenzoyl Hexyl Benzoate) in einer Menge von 0,01 bis 10 Gewichts-%, bezogen auf das Gesamtgewicht der Zubereitung, enthält.

## Claims

1. Use of polysaccharides from the group of gums in cosmetic preparations comprising UV filters for reducing soiling and/or discolouration of textiles caused by the UV filters in the preparation.

2. Use of polysaccharides from the group of gums in cosmetic preparations comprising UV filters for facilitating the ability of the soiling and/or discolouration of textiles caused by the UV filters in the preparation to be washed out.

3. Method for facilitating the ability of cosmetic preparations comprising UV filters to be washed out from textiles, **characterized in that** one or more polysaccharides from the group of gums are added to the cosmetic.

4. Method for reducing the discolouration of textiles caused by cosmetic preparations comprising UV filters, **characterized in that** one or more polysaccharides from the group of gums are added to the cosmetic.

5. Use or method according to any of the preceding claims, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), 4-(tert-butyl)-4'-methoxydibenzoylmethane (INCI: Butyl Methoxydibenzoylmethane), and/or hexyl 2-[4-(diethylamino)-2-hydroxybenzoyl]benzoate (INCI: Diethylamino Hydroxybenzoyl Hexyl Benzoate) as soiling and/or discolouring UV filters.

6. Use or method according to any of the preceding claims, **characterized in that** the polysaccharides used are selected from the group of compounds comprising welan gum, sclerotium gum and cellulose gum.

7. Use or method according to any of the preceding claims, **characterized in that** the preparation comprises polysaccharides in a total amount of 0.01 to 2% by weight, based on the total weight of the preparation.

8. Use or method according to any of the preceding claims, **characterized in that** the preparation comprises one or more acid or salt compounds from the group comprising
- 1-hydroxyethane-(1,1-diphosphonic acid)/HEDP
- aminotrimethylene phosphonic acid/ATMP
- diethylenetriamine penta(methylenephosphonic acid)/DTPMP
- ethylenediamine tetra(methylenephosphonic acid)/EDTMP
- phosphonobutanetricarboxylic acid/PBTC
- iminodisuccinate
- sodium polyphosphate
- tetrasodium pyrophosphate
- hydroxamic acid
- polygalacturonic acid
- succinic acid
- formic acid
- malic acid
- 1-hydroxyethane-(1,1-diphosponic acid)/HEDP
- aminotrimethylene phosphonic acid/ATMP
- diethylenetriamine penta(methylenephosphonic acid)/DTPMP
- ethylenediamine tetra(methylenephosphonic acid)/EDTMP
- phosphonobutanetricarboxylic acid/PBTC
- iminodisuccinate
- sodium polyphosphate
- tetrasodium pyrophosphate
- hydroxamic acid
- polygalacturonic acid
- succinic acid
- formic acid
- malic acid
- ethylenediaminetetraacetic acid (EDTA)
and/or alkali metal salts thereof and/or amine N-oxides thereof.

9. Use or method according to Claim 8, **characterized in that** the preparation comprises one or more of these acid or salt compounds in a total amount 0.1 to 3% by weight, based on the total weight of the preparation.

10. Use or method according to any of the preceding claims, **characterized in that** the preparation comprises one or more siloxane elastomers.

11. Use or method according to Claim 10, **characterized in that** the preparation comprises one or more siloxane elastomers in a total amount of 0.01 to 10% by weight, based on the total weight of the preparation.

12. Cosmetic preparation comprising a combination of
a) 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and
b) welan gum.

13. Cosmetic preparation comprising a combination of
a) 4-(tert-butyl)-4'-methoxydibenzoylmethane (INCI: Butyl Methoxydibenzoylmethane) and
b) welan gum.

14. Cosmetic preparation comprising a combination of
a) hexyl 2-[4-(diethylamino)-2-hydroxybenzoyl]benzoate (INCI: Diethylamino Hydroxybenzoyl Hexyl Benzoate) and
b) welan gum.

15. Cosmetic preparation according to any of Claims 12 to 14, **characterized in that** the total amount of welan gum and sclerotium gum (if present) in the preparation is from 0.01 to 2% by weight, based on the total weight of the preparation.

16. Use, method or preparation according to any of the preceding claims, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) in an amount of 0.01 to 10% by weight, based on the total weight of the preparation.

17. Use, method or preparation according to any of the preceding Claims 1-11, 13 and 15, **characterized in that** the preparation comprises 4-(tert-butyl)-4'-methoxydibenzoylmethane (INCI: Butyl Methoxydibenzoylmethane) in an amount of 0.01 to 10% by weight, based on the total weight of the preparation.

18. Use, method or preparation according to any of the preceding Claims 1 to 11, 14 and 15, **characterized in that** the preparation comprises hexyl 2-[4-(diethylamino)-2-hydroxybenzoyl]benzoate (INCI: Diethylamino Hydroxybenzoyl Hexyl Benzoate) in an amount of 0.01 to 10% by weight, based on the total weight of the preparation.

## Revendications

1. Utilisation de polysaccharides du groupe des gommes dans des préparations cosmétiques contenant des filtres UV, pour réduire les salissures et/ou les colorations de textiles causées par les filtres UV dans la préparation.

2. Utilisation de polysaccharides du groupe des gommes dans des préparations cosmétiques contenant des filtres UV, pour faciliter le lavage des salissures et/ou des colorations de textiles causées par les filtres UV dans la préparation.

3. Procédé visant à faciliter le lavage de préparations cosmétiques contenant des filtres UV sur des textiles, **caractérisé en ce qu'**un ou plusieurs polysaccharides du groupe des gommes sont ajoutés au cosmétique.

4. Procédé visant à réduire les colorations de textiles causées par des préparations cosmétiques contenant des filtres UV, **caractérisé en ce qu'**un ou plusieurs polysaccharides du groupe des gommes sont ajoutés au cosmétique.

5. Utilisation ou procédé selon l'une quelconque des revendications précédentes, caractérisé(e) en ce que la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin), du 4-(tert.-butyl)-4'-méthoxydibenzoylméthane (INCI : Butyl Methoxydibenzoylmethane) et/ou de l'ester hexylique de l'acide (2-[-4-(diéthylamino)-2-hydroxybenzoyl]benzoïque (INCI : Diethylamino Hydroxybenzoyl Hexyl Benzoate) en tant que filtre UV salissant et/ou colorant.

6. Utilisation ou procédé selon l'une quelconque des revendications précédentes, caractérisé(e) en ce que les polysaccharides utilisés sont choisis dans le groupe de composés constitué par la gomme welan, la gomme de sclérotium et la gomme de cellulose.

7. Utilisation ou procédé selon l'une quelconque des revendications précédentes, caractérisé(e) en ce que la préparation contient des polysaccharides en une quantité totale de 0,01 à 2 % en poids, par rapport au poids total de la préparation.

8. Utilisation ou procédé selon l'une quelconque des revendications précédentes, caractérisé(e) en ce que la préparation contient un ou plusieurs composés acides ou sels du groupe constitué par :
- l'acide 1-hydroxyéthane-(1,1-diphosphonique)/ HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- l'iminodisuccinate,
- le polyphosphate de sodium,
- le pyrophosphate de tétrasodium,
- l'acide hydroxamique,
- l'acide polygalacturonique,
- l'acide succinique,
- l'acide formique,
- l'acide malique,
- l'acide 1-hydroxyéthane-(1,1-diphosphonique)/ HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- l'iminodisuccinate,
- le polyphosphate de sodium,
- le pyrophosphate de tétrasodium,
- l'acide hydroxamique,
- l'acide polygalacturonique,
- l'acide succinique,
- l'acide formique,
- l'acide malique,
- l'acide éthylène-diamine-tétraacétique (EDTA),
et/ou leurs sels alcalins et/ou leurs amine-N-oxydes.

9. Utilisation ou procédé selon la revendication 8, caractérisé(e) en ce que la préparation contient un ou plusieurs de ces composés acides ou sels en une quantité totale de 0,1 à 3 % en poids, par rapport au poids total de la préparation.

10. Utilisation ou procédé selon l'une quelconque des revendications précédentes, caractérisé(e) en ce que la préparation contient un ou plusieurs élastomères de siloxane.

11. Utilisation ou procédé selon la revendication 10, caractérisé(e) en ce que la préparation contient un ou plusieurs élastomères de siloxane en une quantité totale de 0,01 à 10 % en poids, par rapport au poids total de la préparation.

12. Préparation cosmétique contenant une combinaison de :
a) de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) et
b) de la gomme welan.

13. Préparation cosmétique contenant une combinaison de :
a) du 4-(tert.-butyl)-4'-méthoxydibenzoylméthane (INCI : Butyl Methoxydibenzoylmethane) et
b) de la gomme welan.

14. Préparation cosmétique contenant une combinaison de :
a) de l'ester hexylique de l'acide (2-[-4-(diéthylamino)-2-hydroxybenzoyl]benzoïque (INCI : Diethylamino Hydroxybenzoyl Hexyl Benzoate) et
b) de la gomme welan.

15. Préparation cosmétique selon l'une quelconque des revendications 12 à 14, **caractérisée en ce que** la quantité totale de gomme welan et de gomme de sclérotium (dans la mesure où elle est contenue) dans la préparation est de 0,01 à 2 % en poids, par rapport au poids total de la préparation.

16. Utilisation, procédé ou préparation selon l'une quelconque des revendications précédentes, caractérisé(e) en ce que la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) en une quantité de 0,01 à 10 % en poids, par rapport au poids total de la préparation.

17. Utilisation, procédé ou préparation selon l'une quelconque des revendications 1 à 11, 13 et 15, caractérisé(e) en ce que la préparation contient du 4-(tert.-butyl)-4'-méthoxydibenzoylméthane (INCI : Butyl Methoxydibenzoylmethane) en une quantité de 0,01 à 10 % en poids, par rapport au poids total de la préparation.

18. Utilisation, procédé ou préparation selon l'une quelconque des revendications 1 à 11, 14 et 15, caractérisé(e) en ce que la préparation contient de l'ester hexylique de l'acide (2-[-4-(diéthylamino)-2-hydroxybenzoyl]benzoïque (INCI : Diethylamino Hydroxybenzoyl Hexyl Benzoate) en une quantité de 0,01 à 10 % en poids, par rapport au poids total de la préparation.
